(19) Europäisches Patentamt
European Patent Office
Office européen des brevets

(11) EP 3 714 940 B1

(12) EUROPEAN PATENT SPECIFICATION

(45) Date of publication and mention of the grant of the patent:
**03.06.2026 Bulletin 2026/23**

(21) Application number: **20164975.3**

(22) Date of filing: **23.03.2020**

(51) International Patent Classification (IPC):
***A61K 36/185*** *(2006.01)* ***A61K 36/57*** *(2006.01)*
***A61K 36/28*** *(2006.01)* ***A61K 36/68*** *(2006.01)*
***A61P 11/14*** *(2006.01)* ***A61K 35/644*** *(2015.01)*
***A61K 9/00*** *(2006.01)* ***A61K 47/12*** *(2006.01)*
***A61K 47/46*** *(2006.01)* ***A61K 47/36*** *(2006.01)*
***A61K 47/26*** *(2006.01)* *A61K 9/08 (2006.01)*
*A61K 9/20 (2006.01)*

(52) Cooperative Patent Classification (CPC):
(C-Sets available)
**A61P 11/14; A61K 9/0095; A61K 35/644; A61K 36/28; A61K 36/57; A61K 36/577; A61K 36/68; A61K 47/12; A61K 47/26; A61K 47/36; A61K 47/46;** A61K 9/08; A61K 9/2068
(Cont.)

# (54) PHARMACEUTICAL FORMULATION FOR ORAL USE CONTAINING HONEY AND PLANT EXTRACTS WITH AN ASSAYED POLYSACCHARIDE AND MUCILAGE CONTENT FOR THE TREATMENT OF PRODUCTIVE AND NON-PRODUCTIVE COUGHS AND THROAT DISEASES

PHARMAZEUTISCHE FORMULIERUNG ZUR ORALEN ANWENDUNG, DIE HONIG UND PFLANZENEXTRAKTE MIT EINEM GETESTETEN POLYSACCHARID- UND SCHLEIMGEHALT ENTHÄLT, ZUR BEHANDLUNG VON PRODUKTIVEM UND NICHT-PRODUKTIVEM HUSTEN UND HALSERKRANKUNGEN

FORMULATION PHARMACEUTIQUE À USAGE ORAL CONTENANT DU MIEL ET DES EXTRAITS VÉGÉTAUX CONTENANT DES POLYSACCHARIDES ET DES MUCILAGES DOSÉS POUR LE TRAITEMENT DE LA TOUX PRODUCTIVE ET NON PRODUCTIVE ET DES MALADIES DE LA GORGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2019 IT 201900004335**

(43) Date of publication of application:
**30.09.2020 Bulletin 2020/40**

(73) Proprietor: **Dr. Willmar Schwabe GmbH & Co. KG**
**76227 Karlsruhe (DE)**

(72) Inventors:
- **RASO, Floriana**
  **39044 EGNA (BZ) (IT)**
- **PANTANO, Doriana**
  **39044 EGNA (BZ) (IT)**

(74) Representative: **Kraus & Lederer PartGmbB**
**Thomas-Wimmer-Ring 15**
**80539 München (DE)**

(56) References cited:
**WO-A1-2013/132456 WO-A1-2018/150309**
**US-A1- 2016 303 179 US-A1- 2016 331 794**

- **WIDY-TYSZKIEWICZ EWA: "Assessment report on Malva sylvestris L. and/or Malva neglecta Wallr., folium and Malva sylvestris L., flos Final", EMA/HMPC/749518/2016 COMMITTEE ON HERBAL MEDICINAL PRODUCTS (HMPC), 20 November 2018 (2018-11-20), pages 1 - 38, XP093051248, Retrieved from the Internet <URL:https://www.ema.europa.eu/en/documents/herbal-report/final-assessment-report-malva-sylvestris-l/malva-neglecta-wallr-folium-malva-sylvestris-l-flos-first-version_en.pdf>**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

(52) Cooperative Patent Classification (CPC): (Cont.)

C-Sets

**A61K 35/644, A61K 2300/00;**
**A61K 36/185, A61K 2300/00;**
**A61K 36/28, A61K 2300/00;**
**A61K 36/57, A61K 2300/00;**
**A61K 36/68, A61K 2300/00**

## Description

The present invention relates to formulations containing honey and plant extracts with an assayed polysaccharide and mucilage content for the treatment of dry and productive cough and throat ailments.

## State of the art

Coughing is an important defence mechanism used by the body to eliminate irritants such as mucus, dust and foreign bodies from the respiratory tract. If coughing continues beyond the limits of the natural reaction to an irritant, it is advisable to consider taking remedies that calm the cough by limiting the irritation of the mucosa, and promote protection of the upper airways.

The classic pharmacological treatments used to treat coughs are based on pharmacological mechanisms, which act on specific receptors.

The drugs indicated for use to treat non-productive (dry) coughs include the following categories:

- Sedative cough medicines which act on the central nervous system by suppressing the cough reflex. This category includes opioids (e.g. codeine) and non-opioids.
- Sedative cough medicines with a peripheral action, which act by blocking the sodium channels and reducing the excitability of nerve conduction at local level. This category includes lidocaine, for example.

However, in the case of a productive cough it is preferable not to suppress the cough reflex, but to promote fluidification of the mucus, which thus becomes more liquid and easier to eliminate.

Medicaments useful for this purpose are classed as:

- Expectorants: which promote the fluid volume of the secretions at bronchial level, making the mucus more fluid.
- Mucolytics: which have a proteolytic action on the mucus; by cleaving the protein bonds, the mucus becomes less viscous and is eliminated more easily from the respiratory tract.

Sedative, mucolytic and expectorant medicaments can trigger various adverse reactions correlated with their use, including dependence in the case of some of said molecules. The use of such medicaments should therefore be avoided where possible.

US 2016/331794 discloses a composition for the treatment of persistent cough comprising polysaccharides of plant origin and honey. WO 2013/132456 discloses a prebiotic mixture including at least one plant polysaccharide fibre, such as mucilages of Malvaceae plant species, and at least one of Propolis and/or Olea europea and/or Aloe vera extracts. US 2016/303179 dislcoses a composition for use in the treatment of cough, comprising as sole active principles Plantago lanceolatum extract, honey, cane sugar, Thymus extract and optionally Sambucus niger juice WO 2018/150309 discloses a composition for the treatment of cough, comprising one or more polysaccharide fractions of plantain, one or more polysaccharide fractions of marshmallow one or more polyphenolic fractions of agrimony, brown sugar and honey.

It has now been found that in solution, oral spray, tablets or syrup, ingredients such as honey or plant extracts (e.g. mallow or plantain), suitably selected and with an assayed polysaccharide and mucilage content, exhibit synergic activity in soothing coughs without suppressing them, and alleviating sore throats, having regard to their important physiological role in protecting the upper airways.

Honey is a viscous sweet ingredient commonly used in the human diet. It consists of a large amount of sugars, mainly dextrose and laevulose, which give the honey characteristics of high density and viscosity, "stickiness", a tendency to absorb moisture from the air, and immunity from some types of deterioration.

Honey has been known since ancient times for its anti-coughing properties, and is one of the main traditional cough remedies, although its action mechanism has not yet been fully clarified.

The honey used in the present invention is a pharmaceutical grade acacia honey. Acacia honey is one the most delicate types of honey, and therefore the best accepted by children. Moreover, its high pharmaceutical quality makes it particularly suitable for medicinal use.

The plant extracts used in the present invention are:

- *Malva sylvestris* mucilages: mallow mucilages consist of a heterogeneous range of polysaccharides, represented by strongly branched structures of D-galactose, L-rhamnose, D-glucuronic and D-galacturonic acids, with the presence of rhamnogalacturonan and arabinogalactan. Taken orally and used in the case of irritated mucosa, they exhibit a protective action due to the presence of bioadhesive and mucilaginous polysaccharides which give rise to the physical formation of a mucin gel that coats the tissue surface.

Mallow, an annual or perennial herbaceous plant belonging to the *Malvaceae* family, originates from Europe and temperate Asia. It grows wild in meadows and waste land in the lowlands. It is traditionally used in disorders of the upper airways because of its emollient activity.

The main constituents of the plant are mucilages, including glucuronic acid, galacturonic acid, rhamnose and galactose; flavonoids; anthocyans, mainly present in the flowers (including malvin and malvidin); vitamins (A, B1, B2 and C); and mineral salts.

- Polysaccharides obtained from *Plantago major, Inula helenium* and *Helichrysum stoechas* Moench: polysaccharides are macromolecules consisting of individual monosaccharides. Polysaccharides have hygroscopic properties and tend to swell and form viscous, highly mucoadhesive gels.

*Plantago major,* a wild herbaceous plant belonging to the *Plantaginaceae* family, grows in uncultivated meadows and fields. It is very common in Europe and Asia, and is traditionally used to treat disorders of the upper airways due to its emollient activity.

*Inula helenium,* an herbaceous perennial with yellow flowers belonging to the *Asteraceae* family, originates from central and western Asia. It promotes an expectorant and antiseptic action.

*Helichrysum stoechas* Moench, a plant belonging to the *Asteraceae* family, is common in Africa, Madagascar, Australia, Europe and Asia. It mainly grows in stony, arid areas and on calcareous hillsides. It has been known since ancient times for its cough-sedative and mucosal decongestant properties.

## Description of the invention

The object of the present invention is therefore a pharmaceutical formulation containing:

a) honey of a pharmaceutically acceptable grade;
*b) Malva sylvestris* mucilage; and
c) a plant extract or a mixture of different plant extracts as defined in the appended claims with a total polysaccharide content ≥30% by weight of the total weight of the dried extract or mixture of extracts.

The honey used in the formulation is preferably acacia honey.

The amount of pharmaceutically acceptable grade honey in the formulation can preferably range between 8% and 40% w/w or between 10% and 40% w/w, more preferably between 15% and 35% w/w, on the total weight of the formulation.

*Malva sylvestris* mucilages are preferably present in an amount ranging between 1% and 10% w/w or between 1% and 6% w/w, more preferably between 1.5% and 4.5% w/w, on the total weight of the formulation.

The extract or mixture of extracts is preferably present in an amount ranging between 1% and 8% w/w, more preferably between 1.5% and 4.5% w/w, on the total weight of the formulation.

The extracts from one or more plants with a total polysaccharide content ≥30% by weight are *Aloe barbadensis, Aloe vera, Helichrysum italicum, Helichrysum stoechas* Moench, *Inula helenium, Plantago major, Matricaria recutita* and *Commiphora myrrha.*

The pharmaceutical formulation according to the invention may contain a further mucoadhesive agent selected from xanthan gum, PVP and hyaluronic acid together with a carrier, diluent, anticaking agent, disintegrating agent, emulsifier, sweetener, gelling agent, preservative, antioxidant, buffering agent, pH adjuster, flavouring agent, trace element and/or mixtures thereof.

A preferred embodiment of the invention is a pharmaceutical formulation comprising:

- acacia honey amounting to 8-30% on the total weight of the formulation;
- *Malva sylvestris* mucilage amounting to 2-10% w/w on the total weight of the formulation;
- a mixture of *Inula helenium, Helichrysum stoechas* Moench and *Plantago major* extracts with a polysaccharide content ≥30%, amounting to 2-8% w/w on the total weight of the formulation.

The pharmaceutical formulation according to the invention is used to treat symptoms associated with dry or productive cough and throat ailments in general, preferably in the form of a solution, oral spray, tablets or syrup, with oral administration and with action on the throat.

The formulations according to the invention can be administered several times a day.

A preferred embodiment of the invention is the formulation containing:

| | |
|---|---|
| Fructose | 30 to 50 g |
| Honey | 20 to 30 g |
| Water | q.s. |
| *Malva sylvestris* mucilages | 1 to 5 g |
| Mixture of plantain, Inula and Helichrysum extracts with a titrated polysaccharide content | 1 to 6 g |
| Dried extract of star aniseed | 0.1 to 1 g |
| Potassium sorbate | 0.1 g |
| Sodium benzoate | 0.1 g |
| Citric acid | 0 to 0.5 g |
| Xanthan gum | 0 to 2 g |

Upper respiratory tract infections (URTIs) are the most common acute disorders seen in doctors' surgeries. Upper respiratory tract infections comprise disorders ranging from the common cold, a mild self-limiting catarrhal syndrome, to potentially fatal diseases. Viruses are involved in most upper respiratory tract infections, but the infection can be bacterial or involve a superinfection, and such situations may require a specific treatment. Rhinitis, pharyngitis, sinusitis, epiglottitis, laryngitis and tracheitis are specific manifestations of said infections. The treatment depends on the underlying cause, and includes symptomatic support, usually with analgesics for headache, sore throat or muscle pains.

The formulations according to the present invention create a protective film on the mucosa of the throat. The functional ingredients they contain include a large amount of polysaccharides and natural mucilages that create a barrier on the oropharyngeal mucosa and the larynx by means of a bioadhesion mechanism. Adherence to the mucosa of the upper respiratory tract limits contact with external irritants, aids hydration and limits irritative processes.

The formulations according to the present invention have an anti-cough activity not mediated by the $\alpha$1 receptor, and can therefore be administered several times a day without any particular side effects.

In particular, the present formulations have a mucoadhesive property that allows the formulation to remain at the site of application to ensure adequate anti-inflammatory, anti-irritant and lubricant efficacy.

The mucoadhesive properties of the formulations according to the present patent have proved significantly superior to similar products present on the market, and have also proved superior in a 1:2 dilution, which is the dilution that best represents the conditions of use in the oral cavity.

It has been demonstrated that to ensure adequate anti-inflammatory, anti-irritant and anti-oedema efficacy, the formulation must remain at the site of application. Adherence of the formulation to the mucosa is called "mucoadhesion".

The ingredients of the formulation according to the present invention act synergically by means of bioadhesion to the mucosa of the pharynx.

The term "mucoadhesion" means a bond between a macromolecule and the epithelial surface of the mucosa.

The adhesion process begins when the dehydrated polymer matrix comes into contact with the surface of the mucosa, and more specifically with the mucosa that covers it. The adhesion process comprises two stages:

1. Contact stage: the polymer is hydrated and establishes contact with the mucosa. At this stage the mucoadhesive substance and the mucosa create close contact between their surfaces. The formulation penetrates the oral cavity and tends to be deposited on the anatomical obstacles present in the respiratory tract. Specifically, contact between the product and the mucosa is guaranteed by the Brownian motion of the polymer particles and by repulsive or attractive forces (van der Waals interactions or hydrogen bonds), depending on whether the polymer has the same charge as the mucosa or the opposite charge. The strength of said bonds naturally varies according to the nature of the particles, the aqueous medium and the distance between the particles and the contact surface. The flow of saliva and the pH of the environment wherein the polymer acts influence the interaction time with the mucosa.
2. Consolidation stage: At this stage a different physicochemical interaction takes place, which consolidates and reinforces the bond between the polymer and the mucosa, leading to lengthy adhesion. The polymer and the mucosal cells generate closer contact and create stronger bonds. The hydration of the mucosa plays a significant part in allowing this bond. On the surface of the respiratory mucosa, where a thinner, non-continuous layer of mucus is present, the dehydrated polymer tends to have a "suction" effect on the water contained in the mucus. As it loses water the mucus tends to collapse, leaving free access to the polymer to create hydrogen bonds with the epithelial cell layer. Moreover, the bond with the polymer reduces the rate of regrowth of the mucosa, and ensures greater persistence of mucoadhesive substances.

The formulation according to the invention has a smoothing, emollient action on the irritated mucosa, known as a "demulcent" action. This term means a soothing and protective action on irritated or inflammatory mucosal tissue. In topical use, the term "demulcent" is replaced by the term "emollient".

The exchange of liquids between the formulation according to the invention and the mucus aids the fluidification of the mucus and its expulsion from the upper respiratory tract.

Moreover the product, due to its viscosity, acts as a lubricant, reducing the friction on the pharynx and larynx that causes coughing.

Optimum mucoadhesion requires a critical degree of hydration. Incomplete hydration due to lack of water leads to incomplete release of the polymer chains. Conversely, an excessive amount of water weakens the mucoadhesive bonds by excessively diluting the polymer solution.

**Example 1 - syrup**

| **Ingredients** | **[% w/w]** |
|---|---|
| Fructose | 40.00 |
| Acacia honey | 30.00 |
| Water | 21.50 |
| *Malva sylvestris* (Mucilage) | 4.00 |
| DEMULTOX ®<br>(mixture of extracts of *Inula helenium, Helichrysum stoechas* Moench and *Plantago major* ≥30% polysaccharide content) | 3.00 |
| Dried extract of star aniseed | 1.00 |
| Essential oil of silver fir | 0.15 |
| Potassium sorbate | 0.10 |
| Sodium benzoate | 0.10 |
| Citric acid | 0.10 |
| Xanthan gum | 0.05 |

**Example 2 - syrup**

| **Ingredients** | **[% w/w]** |
|---|---|
| Fructose | 40.00 |
| Acacia honey | 25.00 |
| Water | 26.65 |
| *Malva sylvestris* (Mucilage) | 4.00 |
| DEMULTOX ®<br>(mixture of extracts of *Inula helenium, Helichrysum stoechas* Moench and *Plantago major* ≥30% polysaccharide content) | 3.00 |
| Dried extract of star aniseed | 1.00 |
| Potassium sorbate | 0.10 |
| Sodium benzoate | 0.10 |
| Citric acid | 0.10 |
| Xanthan gum | 0.05 |

**Example 3 - oral liquid**

| Ingredients | [% w/wl |
|---|---|
| Acacia honey | 20.00 |
| Water | 70.996 |
| *Malva sylvestris* (Mucilage) | 4.00 |
| DEMULTOX ® (mixture of extracts of *Inula helenium, Helichrysum stoechas* Moench and *Plantago major* ≥30% polysaccharide content) | 3.00 |
| Copper | 0.002 |
| Zinc | 0.002 |
| Camomile | 1 |
| Polyvinylpyrrolidone | 1 |

**Example 4 - oral liquid**

| Ingredients | [% w/w] |
|---|---|
| Acacia honey | 20.00 |
| Water | 71.58 |
| *Malva sylvestris* (Mucilage) | 4.00 |
| DEMULTOX ® (mixture of extracts of *Inula helenium, Helichrysum stoechas* Moench and *Plantago major* ≥30% polysaccharide content) | 3.00 |
| Potassium sorbate | 0.1 |
| Sodium benzoate | 0.1 |
| Camomile | 1 |
| Thymus vulgaris | 0.05 |
| Citric acid | 0.05 |
| Steviol glycosides | 0.02 |

**Example 5 - tablet**

| Ingredients | [% w/wl |
|---|---|
| Honey | 8.82 |
| Saccharose | 23.53 |
| *Malva sylvestris* (Mucilage) | 4.00 |
| DEMULTOX ® (mixture of extracts of *Inula helenium, Helichrysum stoechas* Moench and *Plantago major* ≥30% polysaccharide content) | 3.00 |

(continued)

| **Ingredients** | **[% w/wl** |
|---|---|
| Maltodextrins | 16-31% |
| Isomalt | 10-10.30% |
| Camomile | 2.65% |
| Sweeteners (Sucralose or steviol glycosides) | 0-0.08% |
| Citric acid | 1-1.5% |
| Flavourings | q.s. |

**Example 6 - *In vitro* evaluation of mucoadhesion on reconstructed human oral epithelium**

The following study compared the mucoadhesive properties of the formulation described in Example 1 with those of a commercial syrup having a similar composition.

The study demonstrated that the product to which the patent relates has greater mucoadhesive properties than the competing product, even at a 1:2 dilution.

A three-dimensional model of reconstructed human oral epithelium (HOE) tissue based on normal human cells was used to evaluate the *in vitro* mucoadhesion of the products. The mucoadhesion of the products was determined by evaluating the percentage inhibition of the lectin-glycoprotein bond.

Biotinylated lectin (concanavalin-A), a protein contained in some legumes *(Canavalia ensiformis),* which exhibits a high affinity for the glucoside and mannoside residues present in membrane glycoproteins, was used for this purpose. The binding sites are therefore detected with streptavidin peroxidase which, due to its high affinity for biotin, forms a protein-glucose-lectin-biotin-streptavidin-peroxidase complex. The complex is quantified due to the presence of peroxidase, by means of the ortho-phenylenediamine oxidation reaction in the following reaction:

$$\text{O-phenyldiamine} \xrightarrow[\text{Strep. perox.}]{H_2O_2} \text{2,3-diaminophenazine (yellow)}$$

The intensity of the yellow-orange colour of the solution (measured with a spectrophotometer at $\lambda$=450 nm) is proportional to the number of glycoprotein-lectin bonds, and consequently to the number of sites (glycoproteins) available for mucoadhesion. The reduction in the absorbance value compared with the untreated control is proportional to the mucoadhesion capacity of the test substance.

The test was conducted on a three-dimensional model of reconstructed human oral epithelium (HOE) tissue consisting of cells derived from a squamous-cell carcinoma of the oral mucosa cultured on an inert polycarbonate filter at the liquid/air interface in a specific medium. Said model represents an epithelial tissue devoid of stratum corneum, which is histologically similar to the mucosa of the oral cavity.

**Substances tested**

Negative control: sterile deionised water
Positive control: 1% hyaluronic acid
Sample:
TS1 Formulation of Example 1 (as is);
TS2 Formulation of Example 1 (diluted 1:2);
TS3 Commercial syrup having the following qualitative particulars:

| **Ingredient** |
|---|
| Honey |

(continued)

| Ingredient |
|---|
| Molecular complex of resins, polysaccharides and flavonoids obtained from Grindelia, Plantain and Helichrysum (polysaccharide content ≥20%) |
| Sugar cane |
| Water |
| Essential oils of eucalyptus, star aniseed and lemon |
| Natural lemon flavouring |
| Gum arabic |
| Xanthan gum |

**Experimental protocol**

The tissues were left to equilibrate at room temperature for about 15 minutes, then transferred to the growth medium and incubated (37°C, 5% $CO_2$) overnight.

After overnight incubation the tissues were treated for 30 minutes with 70 µl of tested sample or 70 µl of the controls.

Each experiment was conducted in triplicate.

At the end of the treatment period, the tissues were treated in sequence with:

1. Concanavalin A
2. Streptavidin peroxidase
3. o-phenylenediamine + $H_2O_2$

Between one administration and the next, the tissues were repeatedly washed with DPBS.

At the end of the test the optical density (OD) was read at 450 nm.

**Data analysis and calculation of results**

The mucoadhesion was calculated as Mucoadhesion (%) defined as follows:

$$\text{Mucoadhesion (\%) Sample} = (1 - \text{ODSAMPLE} / \text{ODCONTROL}) \times 100$$

The results are set out in Table 1 below.

Table 1

| Sample | Mucoadhesion (%) |
|---|---|
| TS1 | 28.28 |
| TS2 | 23.50 |
| TS3 | 22.40 |

In the tissues treated with sample TS1, mucoadhesion was significantly greater than that of the tissues treated with samples TS2 and TS3.

**Example 7 - *In vitro* evaluation of mucoadhesion on reconstructed human oral epithelium**

The purpose of the study was to evaluate the *in vitro* mucoadhesion of the Formulation of Example 1 and of commercial product C1 towards human buccal cells at two different dilutions (1:2 and 1:5).

A second purpose, developed during the second stage of the study, related to determination, for all three products, of the resistance over time of the mucoadhesive layer (formed by interaction of the product with the buccal mucosa cells) to the action of artificial saliva.

**Substances tested**

**Formulation of Example 1**

**Formulation of Example 1 diluted 1:2**

**Formulation of Example 1 diluted 1:5**

**C1** Commercial syrup having the qualitative particulars set out in the table below

**C1 diluted 1:2**

**C1 diluted 1:5**

| Qualitative particulars of C1 |
|---|
| Acacia honey |
| Complex consisting of extracts of plantain, mallow and Althaea (polysaccharide content > 30%) |
| Helichrysum |
| Grindelia |
| Propolis |
| Water |
| Fructose |
| Strawberry juice |
| Xanthan gum |
| Citric acid |
| Peach and strawberry flavourings |

**1. Evaluation of static mucoadhesion**

The oral cavity of 6 male and female donors (aged 22-42 years), who had not eaten or drunk for at least 60 minutes, was gently scraped with a wooden spatula and the cells were immersed in 0.05M TBS (Tris Buffer Saline) pH 7.6.

After the count with 0.5% Trypan blue, the cells were diluted with 0.9% NaCl to obtain a final concentration of 450,000-480,000 cells per sample to be used in the study. The cells were maintained at the temperature of 4°C.

The cell suspensions were then centrifuged at 2000 rpm for 5 minutes and incubated with 5 ml of the samples to be examined with an aqueous solution containing 0.9% NaCl until a final volume of 5 ml was reached. For the control, the cell suspensions were incubated with 5 ml of an aqueous solution containing 0.9% NaCl.

Incubation continued for 15 minutes at the temperature of 30°C, stirring gently.

3 washes were performed with TBS, the buccal cells were incubated with 5 ml of 10 mg L-1 of Con-A at 30°C for 30 minutes, washed 3 times with TBS and then incubated at 30°C for 60 minutes with 5 ml of 5 mg L-1 of streptavidin peroxidase.

After 3 washes with TBS, 240,000 cells per sample were added to 2.5 ml of o-phenylenediamine (o-pd) in 0.05M phosphate citrate and H2O2, and the reaction was quenched with 1M H2SO4 after 5 minutes.

The absorbance values of each determination were then read with a spectrophotometer. As the products to be evaluated (Formulation of Example 1 and C1) have a colour which could interfere with correct determination of the absorbance value recorded at the end of the protocol, a NaCl solution (0.9%) was used as control for reading of the samples, and the solution of the product, suitably diluted with the 0.9% NaCl solution, was used as blank.

The evaluation was repeated three times for each product, and the results therefore represent the means $\pm$ SEM of all the experiments conducted.

**2. Evaluation of dynamic mucoadhesion retention.**

A system consisting of six Franz cells was used for this study. Franz cells are widely used in the literature to study

the process of absorption of a compound through the skin or other artificial or biological membranes.

The Franz cell consists of a donor and a receptor (Fig. 1).

In the experiment conducted to evaluate the mucoadhesion retention of the formulation tested, buccal cell cultures, obtained as described in the first experimental phase, were placed in the donor and treated with the formulations reported above.

Of the dilution ratios examined in the first phase of the study (1:2 and 1:5), the ratio 1:2 was selected to evaluate mucoadhesion resistance because it was considered to be the closest to the real conditions that may arise *in vivo.*

The donor was fed with a continuous flow (2 ml/min.) of artificial saliva solution, consisting of an isotonic aqueous solution containing a pH=7 phosphate buffer and 0.5% mucin.

A six-channel peristaltic pump able to serve the entire set (six) of Franz cells was used for the circulation of the artificial saliva solution.

The saliva solution was thermostated at the temperature of 36°C to simulate the application conditions of the product *in vivo* as closely as possible.

A cellulose acetate membrane designed to allow the outflow of the saliva solution from donor to receptor, while retaining the mucosal cells in the donor, was placed at the base of the donor, in the zone of separation from the receptor.

Initially, the receptor was filled with artificial saliva solution, thermostated at the temperature of 36°C using the water jacket present in the Franz cell.

The experiment regarding the flow of saliva through mucosal cells treated with the formulation examined was interrupted at various times: after 0.5, 1 and 2 hours.

At this point, the donor cells were transferred to a test tube and treated firstly with biotinylated lectin (Con-A) and secondly, following the same experimental scheme as used in the first phase, with streptavidin peroxidase in the presence of ortho-phenylenediamine, to determine the residual mucoadhesion percentage after treatment with saliva solution at the different times.

Each experiment, relating to a given time of exposure to the flow of the saliva solution, was conducted in triplicate.

## RESULTS

### 1. Static mucoadhesion

The percentages of mucoadhesion to human buccal cells of the "as is" and diluted (1:2 and 1:5) samples obtained in this study are shown in Table 2 below and in the chart in Figure 2.

Table 2

| | Mucoadhesion % Undiluted sample | Mucoadhesion % Sample diluted 1:2 | Mucoadhesion % Sample diluted 1:5 |
|---|---|---|---|
| **Sample C 1** | | | |
| A | 57.5 | 46.4 | 38.7 |
| B | 48.4 | 38.5 | 34.8 |
| C | 52.8 | 43.2 | 30.2 |
| *Mean ± S.D.* | *52.9 ± 4.5* | *42.7 ± 4.0*** | *34.6 ± 4.2**** |
| **Formulation of Example 1** | | | |
| A | 71.5 | 63.5 | 51.3 |
| B | 62.8 | 58.4 | 46.8 |
| C | 65.7 | 56.7 | 53.6 |
| *Mean ± S.D.* | *66.7 ± 4.4* | *59.5 ± 3.5 ** | *50.6 ± 3.5**** |
| *Mean ± S.D.* | *72.1 ± 3.4* | *57.3 ± 5.1*** | *49.5 ± 2.9**** |
| Undiluted versus diluted (1:2 or 1:5): *p>0.05, **p<0.05; ***p<0.01<br>p*>0.05 (not significantly different), p** <0.05 (fairly significant difference), p***<0.01 (highly significant difference); | | | |

wherein p is the level of significance assigned. A threshold value, generally 0.05, having been assigned:

1) $p>0.05$ values not significantly different
2) $p<0.05$ but $>0.01$ difference between values fairly significant
3) $p<0.01$ difference between values highly significant

Thus the lower the p-value, the more significant the difference between the values measured.

Comparison between:
Formulation of Example 1 and Product C1: Undiluted $p<0.05$; Diluted 1:2 $p<0.01$; Diluted 1:5, $p<0.01$

The results indicate significant mucoadhesive activity for the Formulation of Example 1, both when the samples were used "as is" (undiluted products) and when they were diluted 1:2 and 1:5 with saline solution.

C1 exhibits modest mucoadhesive properties compared with the Formulation of Example 1.

Moreover, the mucoadhesion of the Formulation of Example 1 does not decline significantly ($p>0.05$) between the undiluted sample and the 1:2 dilution. Conversely, the mucoadhesion of C1 declines significantly ($p=0.01$; $p<0.01$) for the 1:2 and 1:5 dilutions respectively.

The low standard deviation values denote not only the validation, but also the reproducibility of the model.

The product was diluted (1:2 and 1:5) with saline solution to simulate as closely as possible the real conditions of use of the product which, after being taken, is diluted by the saliva.

However, of the two dilutions, the 1:2 dilution can reasonably be considered the closest to the conditions of use which take place after administration of the product.

Conversely, the comparison with C1 shows significantly greater mucoadhesion of the Formulation of Example 1 at all dilutions.

The Formulation of Example 1 therefore exhibits high mucoadhesion to the mucosal cells, thus giving rise to high coverage and consequently protection of the mucosal surface.

**2. Dynamic mucoadhesion: resistance of the mucoadhesive layer to the action of a simulated saliva solution:**

As regards the second phase of the study evaluating the resistance of the mucoadhesive layer (formed by interaction between the ingredients contained in the product and the mucosal cells), the 1:2 dilution was used for each product, as this dilution is the one that most closely simulates the experimental conditions that arise after application of the product to the mucosa.

The results obtained, set out in Table 3, indicate an interesting ability of said ingredients to bioadhere to the mucosa for a period compatible with the performance of the protective function exercised by the products after application to the mucosa.

The mucoadhesion value remained sufficiently detectable even two hours after application of the product, albeit to a lesser extent, as can be seen from Table 2.

Table 3 shows the resistance of the mucoadhesive layer obtained with the Formulation of Example 1 and C1 diluted 1:2 at various times (0.5 h, 1 h and 2h) vs a simulated saliva solution (saline sol., 0.9% NaCl).

Table 3

| Samples | Diluted 1:2 (0 hours) | 0.5 h | 1 h | 2 h |
|---|---|---|---|---|
| **Product C1** | | | | |
| A | 46.4 | 43.7 | 40.5 | 34.5 |
| B | 38.5 | 34.5 | 32.1 | 26.4 |
| C | 43.2 | 40.2 | 36.7 | 31.6 |
| Mean ± S.D. | *42.7 ± 4.0* | *40.7 ± 6.4** | *36.4 ± 4.2** | *30.8 ± 4.1*** |
| P | | *0.670* | *0.133* | *0.023* |
| **Formulation of Example 1** | | | | |
| A | 63.5 | 61.6 | 54.8 | 46.2 |
| B | 58.4 | 55.4 | 51.6 | 48.3 |
| C | 56.7 | 53.1 | 50.5 | 43.5 |
| Mean ± S.D. | *59.5 ± 3.5* | *56.7 ± 4.4** | 52.3 ± 2.3** | *46.0 ± 2.4**** |

(continued)

| Formulation of Example 1 | | | | |
|---|---|---|---|---|
| P | | *0.437* | *0.041* | *0.005* |
| Undiluted versus diluted (1:2 or 1:5): *p>0.05, **p<0.05; ***p<0.01<br>p*>0.05 (not significantly different), p** <0.05 (fairly significant difference), p***<0.01 (highly significant difference); | | | | |

wherein p is the level of significance assigned.

Comparison between the 3 samples diluted 1:2 at t=0; t=0.5; t=1 h; t=2 h:
Formulation of Example 1 and Product C1: t=0 p=0.005 p<0.01; t=0.5 h p=0.023 p<0.05; t=1 h p=0.005 p<0.01; t=2 h p=0.005 p<0.01

Figure 3 shows the resistance of the mucoadhesive layer obtained with the Formulation of Example 1 and C1 diluted 1:2 at various times (0.5 h, 1 h and 2h) vs a simulated saliva solution (saline sol., 0.9% NaCl).

**CONCLUSIONS**

The results obtained in this study demonstrate that the Formulation of Example 1 performs an evident mucoadhesive activity towards human mucosal cells.

Of the two products evaluated, the Formulation of Example 1 exhibited a high mucoadhesive capacity, generating a mucoadhesive layer sufficiently resistant (dynamic mucoadhesion) to the action of the mucosal fluids with which it comes into contact. This characteristic, namely the formation of a compact, and above all more resistant mucosal layer, leads to longer residence time of the product on the mucosa, and therefore a greater and lengthier protective action.

The data obtained, especially for the Formulation of Example 1, indicate a high level of ability to retain mucoadhesion during the first and second hour of application.

In view of the results obtained, it can be concluded that the Formulation of Example 1, which gives rise to a high level of resistant mucoadhesion, is able to play an effective part in the protective treatment of the oropharyngeal tract, increasing both the efficacy and the duration of action of the treatment.

**Claims**

1. Pharmaceutical formulation containing:

    a) pharmaceutically acceptable grade honey;
    b) *Malva sylvestris* mucilage; and
    c) extract of a plant or a mixture of extracts of different plants with a total polysaccharide content ≥30% by weight on the total weight of the extract or mixture of dried extracts

    wherein the extracts of one or more plants are extracts of *Aloe barbadensis, Aloe vera, Helichrysum italicum, Helichrysum stoechas* Moench, *Inula helenium, Plantago major, Matricaria recutita* or *Commiphora myrrha.*

2. The pharmaceutical formulation according to claim 1 wherein the pharmaceutically acceptable grade honey is acacia honey.

3. The pharmaceutical formulation according to claim 1 or 2 wherein the pharmaceutically acceptable grade honey is present in an amount ranging between 8% w/w and 40% w/w on the total weight of the formulation.

4. The pharmaceutical formulation according to claim 3 wherein the pharmaceutically acceptable grade honey is present in an amount ranging between 15% w/w and 35% w/w on the total weight of the formulation.

5. The pharmaceutical formulation according to each of the previous claims wherein the Malva mucilage is present in an amount ranging between 1% w/w and 10% w/w on the total weight of the formulation.

6. The pharmaceutical formulation according to claim 5 wherein the Malva mucilage is present in an amount ranging between 1.5% w/w and 4.5% w/w on the total weight of the formulation.

7. The pharmaceutical formulation according to each of the preceding claims wherein the extract or mixture of extracts is

present in an amount ranging between 1% and 8% w/w on the total weight of the formulation.

**8.** The pharmaceutical formulation according to claim 7 wherein the extract or the mixture of extracts is present in an amount ranging between 1.5% w/w and 4.5% w/w on the total weight of the formulation.

**9.** The pharmaceutical formulation according to each of the previous claims containing a further mucoadhesive agent selected from xanthan gum, PVP and hyaluronic acid.

**10.** The pharmaceutical formulation according to each of the preceding claims, further containing a carrier, diluent, anticaking agent, disintegrating agent, emulsifier, sweetener, gelling agent, preservative, antioxidant, buffering agent, pH adjuster, flavouring agent, trace element and/or mixtures thereof.

**11.** The pharmaceutical formulation according to claim 1 comprising:

- honey amounting to 8-30% w/w on the total weight of the formulation;
- *Malva sylvestris* mucilage amounting to 2-10% w/w on the total weight of the formulation;
- a mixture of *Inula helenium, Helichrysum stoechas* Moench and *Plantago major* extracts with a polysaccharide content ≥30%, amounting to 2-8% w/w on the total weight of the formulation.

**12.** The pharmaceutical formulation according to each of the previous claims in the form of a solution, oral spray, tablet or syrup.

**13.** The pharmaceutical formulation according to each of the preceding claims for oral use in the treatment of dry and productive cough and throat ailments.

**Patentansprüche**

**1.** Pharmazeutische Formulierung, enthaltend:

a) Honig von pharmazeutisch annehmbarer Qualität;
b) Schleim von *Malva sylvestris;* und
c) Extrakt einer Pflanze oder ein Gemisch von Extrakten verschiedener Pflanzen mit einem Gesamtpolysaccharidgehalt ≥30 Gew.-%, bezogen auf das Gesamtgewicht des Extraktes oder des Gemisches von getrockneten Extrakten,

wobei die Extrakte einer oder mehrerer Pflanzen Extrakte von *Aloe barbadensis, Aloe vera, Helichrysum italicum, Helichrysum stoechas* Moench, *Inula helenium, Plantago major, Matricaria recutita* oder *Commiphora myrrha* sind.

**2.** Pharmazeutische Formulierung nach Anspruch 1, wobei der Honig von pharmazeutisch annehmbarer Qualität Akazienhonig ist.

**3.** Pharmazeutische Formulierung nach Anspruch 1 oder 2, wobei der Honig von pharmazeutisch annehmbarer Qualität in einer Menge im Bereich zwischen 8% Gew./Gew. und 40% Gew./Gew., bezogen auf das Gesamtgewicht der Formulierung, vorliegt.

**4.** Pharmazeutische Formulierung nach Anspruch 3, wobei der Honig von pharmazeutisch annehmbarer Qualität in einer Menge im Bereich zwischen 15% Gew./Gew. und 35% Gew./Gew., bezogen auf das Gesamtgewicht der Formulierung, vorliegt.

**5.** Pharmazeutische Formulierung nach einem der vorhergehenden Ansprüche, wobei der Malva-Schleim in einer Menge im Bereich zwischen 1% Gew./Gew. und 10% Gew./Gew., bezogen auf das Gesamtgewicht der Formulierung, vorliegt.

**6.** Pharmazeutische Formulierung nach Anspruch 5, wobei der Malva-Schleim in einer Menge im Bereich zwischen 1,5% Gew./Gew. und 4,5% Gew./Gew., bezogen auf das Gesamtgewicht der Formulierung, vorliegt.

**7.** Pharmazeutische Formulierung nach einem der vorhergehenden Ansprüche, wobei der Extrakt oder das Gemisch

von Extrakten in einer Menge im Bereich zwischen 1% und 8% Gew./Gew., bezogen auf das Gesamtgewicht der Formulierung, vorliegt.

**8.** Pharmazeutische Formulierung nach Anspruch 7, wobei der Extrakt oder das Gemisch von Extrakten in einer Menge im Bereich zwischen 1,5% Gew./Gew. und 4,5% Gew./Gew., bezogen auf das Gesamtgewicht der Formulierung, vorliegt.

**9.** Pharmazeutische Formulierung nach einem der vorhergehenden Ansprüche, enthaltend ein weiteres mukoadhäsives Mittel, ausgewählt aus Xanthangummi, PVP und Hyaluronsäure.

**10.** Pharmazeutische Formulierung nach einem der vorhergehenden Ansprüche, weiterhin enthaltend einen Träger, ein Verdünnungsmittel, ein Antibackmittel, ein Zerfallsmittel, einen Emulgator, einen Süßstoff, ein Geliermittel, ein Konservierungsmittel, ein Antioxidationsmittel, ein Puffermittel, ein Mittel zur Einstellung des pH, einen Aromastoff, ein Spurenelement und/oder Gemische davon.

**11.** Pharmazeutische Formulierung nach Anspruch 1, umfassend:

- Honig in einer Menge von 8-30% Gew./Gew., bezogen auf das Gesamtgewicht der Formulierung;
- *Malva sylvestris-Schleim* in einer Menge von 2-10 % Gew./Gew., bezogen auf das Gesamtgewicht der Formulierung,
- ein Gemisch aus *Inula helenium-, Helichrysum stoechas* Moench- und *Plantago* major-Extrakten mit einem Polysaccharidgehalt ≥30%, in einer Menge von 2-8% Gew./Gew., bezogen auf das Gesamtgewicht der Formulierung.

**12.** Pharmazeutische Formulierung nach einem der vorhergehenden Ansprüche in Form einer Lösung, eines oralen Sprays, einer Tablette oder eines Sirups.

**13.** Pharmazeutische Formulierung nach einem der vorhergehenden Ansprüche zur oralen Anwendung bei der Behandlung von trockenem und produktivem Husten und Rachenbeschwerden.

**Revendications**

**1.** Formulation pharmaceutique contenant :

a) du miel de qualité pharmaceutiquement acceptable ;
b) du mucilage de *Malva sylvestris ;* et
c) extrait d'une plante ou mélange d'extraits de différentes plantes ayant une teneur totale en polysaccharides ≥ 30 % en poids par rapport au poids total de l'extrait ou du mélange d'extraits secs, les extraits d'une ou plusieurs plantes étant des extraits *d'Aloe barbadensis, d'Aloe vera, d'Helichrysum italicum, d'Helichrysum stoechas* Moench, *d'Inula helenium, de Plantago major, de Matricaria recutita* ou de *Commiphora myrrha.*

**2.** Formulation pharmaceutique selon la revendication 1, dans laquelle le miel de qualité pharmaceutiquement acceptable est du miel d'acacia.

**3.** Formulation pharmaceutique selon la revendication 1 ou 2, dans laquelle le miel de qualité pharmaceutiquement acceptable est présent en une quantité comprise entre 8 % p/p et 40 % p/p du poids total de la formulation.

**4.** Formulation pharmaceutique selon la revendication 3, dans laquelle le miel de qualité pharmaceutiquement acceptable est présent en une quantité comprise entre 15 % p/p et 35 % p/p du poids total de la formulation.

**5.** Formulation pharmaceutique selon chacune des revendications précédentes, dans laquelle le mucilage de *Malva* est présent en une quantité comprise entre 1 % p/p et 10 % p/p du poids total de la formulation.

**6.** Formulation pharmaceutique selon la revendication 5, dans laquelle le mucilage de *Malva* est présent en une quantité comprise entre 1,5 % p/p et 4,5 % p/p du poids total de la formulation.

**7.** Formulation pharmaceutique selon chacune des revendications précédentes, dans laquelle l'extrait ou le mélange

d'extraits est présent en une quantité comprise entre 1 % et 8 % p/p du poids total de la formulation.

**8.** Formulation pharmaceutique selon la revendication 7, dans laquelle l'extrait ou le mélange d'extraits est présent en une quantité comprise entre 1,5 % p/p et 4,5 % p/p du poids total de la formulation.

**9.** Formulation pharmaceutique selon chacune des revendications précédentes, contenant un autre agent mucoadhésif choisi parmi la gomme xanthane, le PVP et l'acide hyaluronique.

**10.** Formulation pharmaceutique selon chacune des revendications précédentes, contenant en outre un excipient, un diluant, un agent anti-agglomérant, un agent désintégrant, un émulsifiant, un édulcorant, un gélifiant, un conservateur, un antioxydant, un agent tampon, un régulateur de pH, un agent aromatisant, un oligo-élément et/ou des mélanges de ceux-ci.

**11.** Formulation pharmaceutique selon la revendication 1 comprenant :

- du miel représentant 8 à 30 % en poids par rapport au poids total de la formulation ;
- du mucilage de *Malva sylvestris* représentant 2 à 10 % p/p du poids total de la formulation ;
- un mélange d'extraits *d'Inula helenium, d'Helichrysum stoechas* Moench et de *Plantago major* avec une teneur en polysaccharides $\geq$ 30 %, représentant 2 à 8 % en poids par rapport au poids total de la formulation.

**12.** Formulation pharmaceutique selon chacune des revendications précédentes sous forme de solution, de spray buccal, de comprimé ou de sirop.

**13.** Formulation pharmaceutique selon chacune des revendications précédentes pour une utilisation orale dans le traitement de la toux sèche et productive et des affections de la gorge.

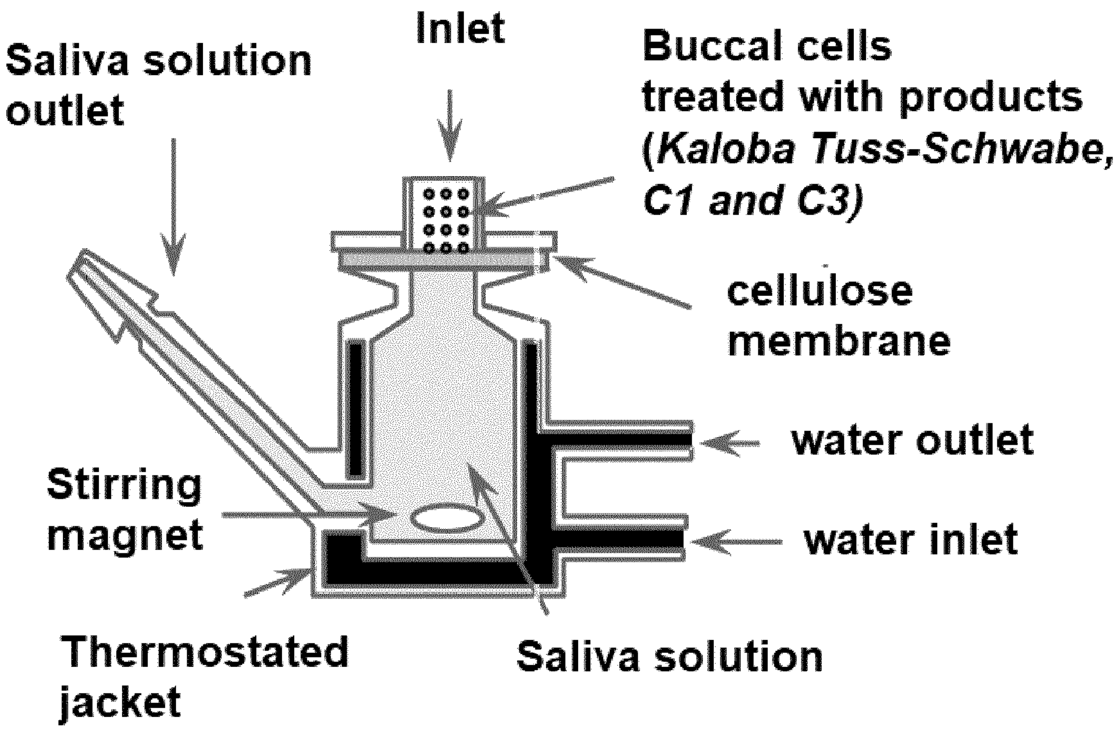
Saliva solution outlet
Inlet
Buccal cells treated with products (*Kaloba Tuss-Schwabe, C1 and C3)*
cellulose membrane
water outlet
water inlet
Stirring magnet
Thermostated jacket
Saliva solution

Figure 1

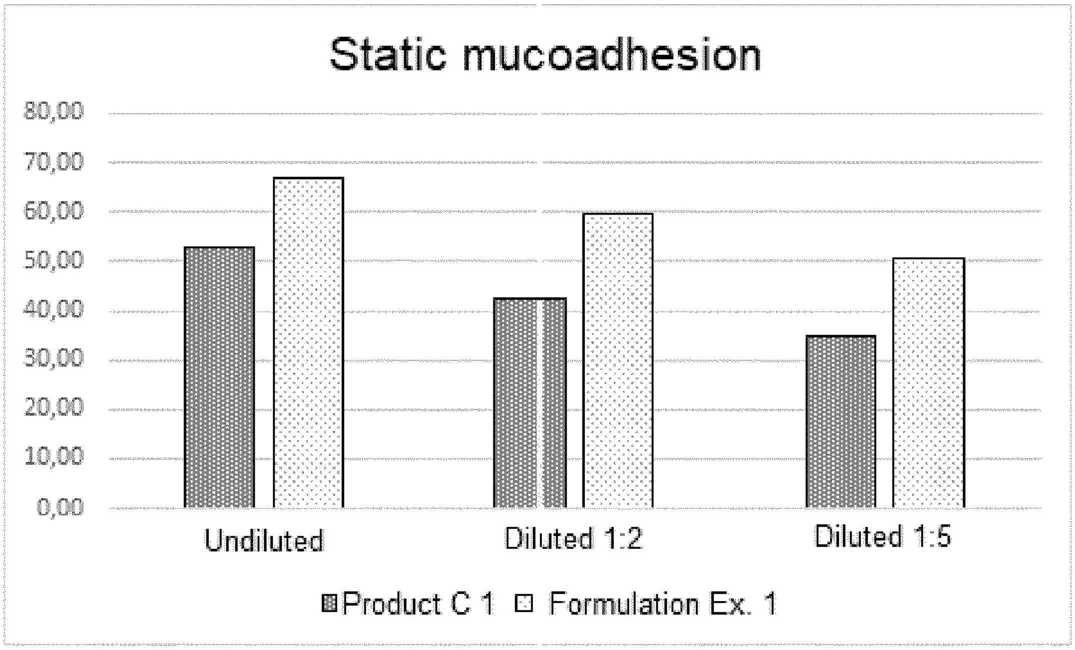
Static mucoadhesion
80,00
70,00
60,00
50,00
40,00
30,00
20,00
10,00
0,00
Undiluted
Diluted 1:2
Diluted 1:5
Product C 1
Formulation Ex. 1

Figure 2

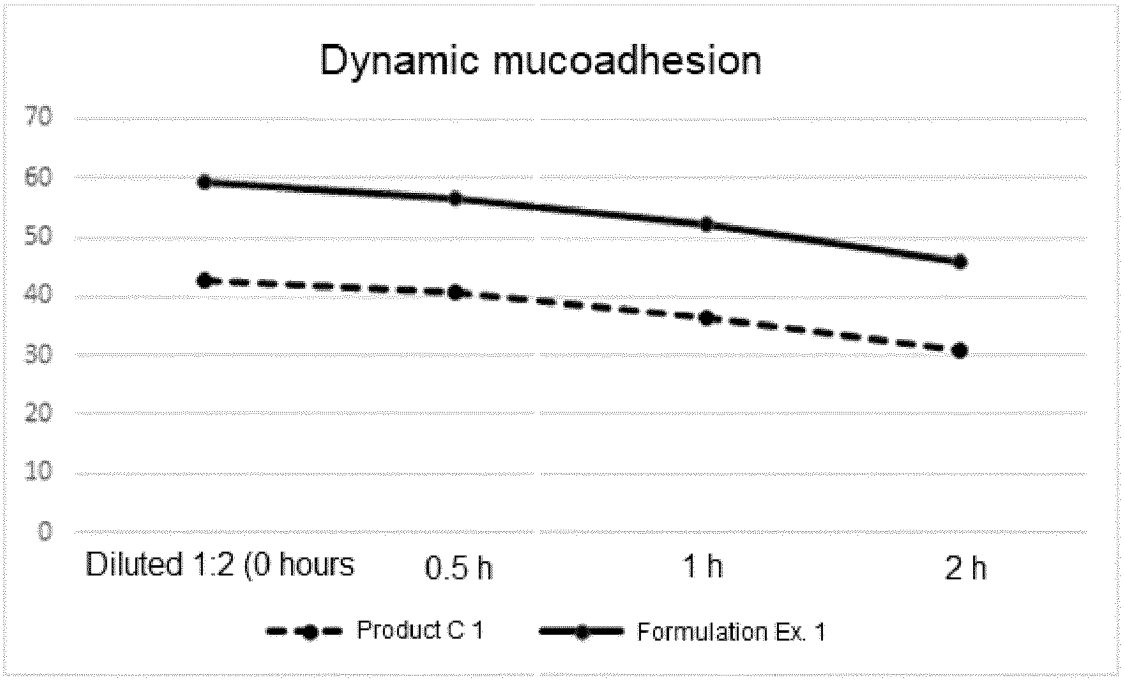
Dynamic mucoadhesion
70
60
50
40
30
20
10
0
Diluted 1:2 (0 hours
0.5 h
1 h
2 h
Product C 1
Formulation Ex. 1

**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016331794 A
- WO 2013132456 A
- US 2016303179 A
- WO 2018150309 A